# EUROPEAN PATENT APPLICATION

(11) **EP 4 717 202 A2**
(43) Date of publication of application: **01.04.2026**
(21) Application number: 26158852.9
(22) Date of filing: 22.08.2023
(51) Int. Cl.: A61B 17/70

(54) **SYSTEMS AND METHODS FOR SECURELY ATTACHING AN ANCHOR TO AN IMPLANT BODY**

(30) Priority: 22.08.2022 US 202263400035 P
(62) Divisional of application: 23858005.4
(71) Applicant: Nexus Spine, LLC, Salt Lake City, Utah 84121 (US)
(72) Inventor: The designation of the inventor has not yet been filed
(74) Representative: Dummett Copp LLP

(57) **Abstract**

Systems and methods for providing a secure and durable implant are disclosed. For example, the systems and methods may include securely attaching an anchor to an implant body. In some cases, the anchor has an anchor head. In some cases, a coupler for coupling to the implant body is configured to receive the anchor. Some implementations of the disclosed systems and methods include a rivet for attaching the anchor to the coupler. In some implementations at least one of the anchor head and the rivet includes a projection, while the other includes a socket configured to receive the projection. Other implementations are discussed herein.

## Description

### FIELD

The present disclosure relates to implants, and more particularly to systems and methods for reducing loosening and separation between an anchor head and an implant body.

### BACKGROUND AND RELATED ART

A wide variety of medical procedures require the use of medical implants. While there are many different kinds of medical implants for treating various medical conditions, many of these implants share a common characteristic of being anchored to the patient's body in some manner. Accordingly, many implants have a need for implant fasteners, such as press-fit implants, tapered implants, cemented implants, threaded implants, and others.

One mechanism commonly used in connection with implants in a medical procedure is a set screw system. In a traditional set screw system, a screw head is retained within a coupler through use of a set screw (typically a smaller screw), which may be screwed into the coupler, the screw head, or another portion of the system.

Unfortunately, in a traditional system, the act of tightening a set screw causes potential energy to be stored in the tightened construct. The set screw is often retained in its tightened, high-energy position only through friction. Accordingly, when the system is exposed to external forces (e.g., vibration, loading, interaction with the system's environment, etc.), the stored potential energy in the set screw may be released, causing the set screw to loosen and compromising the integrity of the entire system (e.g., creating a risk of the screw head becoming detached from the coupler, or decreasing the rigidity and fidelity of the screw). Similar instances of loosening have been observed in many types of implants, wherever set screws are used.

Set screw loosening can not only reduce the effectiveness, durability, efficiency, and comfort of medical implants, but it could also be dangerous to patients, resulting in lasting injury, additional surgery, or even death.

For at least these reasons, there are significant limitations to the current technology in the implant industry. Accordingly, it would be an improvement in the art to augment or even replace current techniques with other techniques.

### BRIEF SUMMARY

Systems and methods for providing a secure and durable implant are disclosed. For example, the systems and methods may include securely attaching an anchor to an implant body. In some cases, the anchor has an anchor head. In some cases, a coupler for coupling to the implant body is configured to receive the anchor. Some implementations of the disclosed systems and methods include a rivet for attaching the anchor to the coupler. In some implementations, at least one of the anchor head and the rivet includes a projection, while the other includes a socket configured to receive the projection. Other implementations are discussed herein.

In some implementations of the disclosed systems and methods, the anchor comprises a shaft. In some cases, the shaft is configured to attach to a body of a patient; as an example, in some implementations, the shaft is threaded.

In some instances, the rivet has a rivet base configured to remain exterior to the coupler (e.g., by abutting a portion of the exterior of the coupler while another portion of the rivet is inserted into the coupler). The rivet has, in some implementations, a rivet neck configured to extend through at least a portion of the coupler. In some implementations, the rivet base also has a rivet port (or multiple rivet ports) for receiving a portion of an applicator configured to assist a user in coupling a rivet head to the anchor through the coupler. Some implementations of the rivet head include a collar for strengthening a connection between the rivet and the coupler.

According to some implementations, the rivet head has multiple rivet head members (e.g., two, three, four, or more members). In some cases, the rivet head has an expanded configuration and a compressed configuration (and may have other configurations as well). As an example, in some implementations, when the rivet head is in the expanded configuration some or all of the rivet head members are separated from each other by a gap, and when the rivet head is in the compressed configuration, some or all of the rivet head members are touching or abutting each other, or they are closer together (i.e., the gap is smaller) than when the rivet head is in the expanded configuration.

In some implementations, the socket formed in the anchor head has an opening (e.g., through which the socket may be accessed). In some cases, the rivet head has a size and shape such that rivet head does not fit through the opening when the rivet head is in the expanded configuration. In some cases, the rivet head does fit through the opening when the rivet head is in the compressed configuration. Accordingly, by moving the rivet head from the expanded configuration to the compressed configuration, this (in some cases) allows the rivet head to be inserted into or removed from the socket. In some implementations, the rivet head members are configured such that the rivet head is biased toward the expanded configuration (e.g., the rivet head is in the expanded configuration when the rivet head members are in a default or relaxed state).

In some implementations, the disclosed systems and methods include a plug that can be inserted into the rivet. In some cases, when the plug is inserted into the rivet, the rivet head is retained in the expanded configuration. Although the plug can have any suitable configuration, in some cases, the plug has a plug body and a plug head. In some such cases, the plug head is configured to retain the rivet head in the expanded configuration when the plug head is positioned within a first cavity of the rivet head. Moreover, in some cases, the rivet head includes a second cavity (e.g., proximate the first cavity), and when the plug head is positioned within the second cavity, the plug does not prevent the rivet head from shifting to the compressed configuration. Thus, the plug, in some cases, locks the rivet head in the expanded configuration when the plug head is in the first cavity, but does not lock the rivet head (leaving it free to move between the expanded configuration and the compressed configuration) when the plug head is positioned within the second cavity (or when the plug is not inserted into the rivet head at all). As an example, in some configurations, the plug head can be moved from the first cavity to the second cavity by inserting the plug farther into the rivet head.

Some implementations of the plug include a plug end. In some cases, the plug end prevents the plug from being inserted too far into the rivet head (for example, the plug end may be too large to fit within the rivet head). In some cases, the plug end merely makes it more difficult to insert the plug past a certain point (e.g., causing the plug to naturally stop when it is inserted to the point that the plug head is disposed within the first chamber); but wherein application of additional force can move the plug end past the stopping point (e.g., pushing the plug head into the second chamber). In some instances, the plug includes one or more ridges that make it easier to insert the plug to a desired position, or that provide tactile feedback to a practitioner applying the plug.

In some implementations, instead of (or in addition to) forming a protrusion configured to be received by a socket in the anchor head, the rivet head itself includes a socket. In some such cases, the anchor head includes a protrusion configured to be received within the socket of the rivet head. Accordingly, the anchor head may include any of the features of the rivet head and the rivet head may include any of the features of the anchor head (as discussed herein).

In some implementations, the disclosed systems and methods include an applicator. In some cases, the applicator is configured to assist a user in coupling the rivet head to the anchor (e.g., through the coupler). For example, some implementations of the applicator are configured to selectively secure the applicator to the rivet (e.g., so that a practitioner can hold the applicator with the rivet attached to the end, apply the rivet, and release the rivet from the applicator once it is applied). In some cases, the applicator includes a retainer that selectively secures the applicator to the rivet. Some instances of the applicator include an actuator configured to insert (or assist a practitioner in inserting) the plug into the rivet head. For example, in some cases, by pushing on a user end of the applicator, the other end-a rivet end of the actuator-may be forced in the direction of the push, consequently pushing on the plug and inserting it into the desired position in the rivet head.

Some implementations of the applicator include a casing. The casing, in some cases, houses other portions of the actuator, such as the retainer or the actuator. The applicator also has, in some instances, additional features, such as an applicator body. Some implementations of the applicator body house the actuator or include a stabilizer that is configured to be inserted into a port of the rivet base (thereby more securely holding the rivet). Some implementations include a retaining anchor (e.g., as part of the retainer), which can selectively connect to the rivet (for example, by attaching to a rivet port, such as by being inserted through a rivet port and latching on by way of a friction fit, an interference fit, or any other suitable attachment mechanism). In some implementations, any or all of the retainer, the applicator body, and the actuator are independently slidable within the applicator casing, thus allowing any or each to be manipulated independently of the others (e.g., disconnecting the retainer, withdrawing the actuator while the retainer is still connected, etc.).

Some implementations of the disclosed systems and methods include a method of securing an anchor to an implant body. In some cases, the method includes one or more of the following: obtaining a coupler configured to couple to the implant body; inserting an anchor head of the anchor into the coupler; and inserting a rivet head of a rivet into a socket of the anchor head, thereby attaching the anchor to the coupler. As the systems and methods disclosed herein are compatible with one another, the systems discussed herein can be used in practicing the methods disclosed herein, and vice versa. For example, in some cases, the rivet head has an expanded configuration, and the rivet head is configured to remain securely within the socket when the rivet head is in the expanded configuration. In some implementations, the method includes inserting a plug into the rivet to selectively lock the rivet head in the expanded configuration.

In some implementations, the method includes activating an actuator of an applicator, such as to insert the plug into the rivet. In some cases, the actuator is configured to selectively couple to a base of the rivet. In some instances, the securing the anchor to a patient is performed prior to inserting the rivet head into the socket, whereas in some cases it is performed concurrently with or after inserting the rivet head into the socket.

Generally speaking, the systems disclosed herein can be made in any suitable manner, and they may be used in any way consistent with their operational capabilities. Moreover, in some cases, any particular element or elements of any apparatus-or portion or portions of any method-disclosed herein can be omitted.

### BRIEF DESCRIPTION OF THE DRAWINGS

The objects and features of the present disclosure will become more fully apparent from the following description and appended claims, taken in conjunction with the accompanying drawings. Understanding that these drawings depict only typical embodiments of the disclosed systems and methods, are not necessarily drawn to scale or in proper proportion, and are, therefore, not to be considered limiting of its scope, the systems and methods will be described and explained with additional specificity and detail through the use of the accompanying drawings in which:
FIG. **1** shows a perspective view of a set screw system, in accordance with some prior art methods of attaching a screw to an implant rod;
FIG. **2**A shows an elevation view of a rivet system for attaching an anchor to an implant body, in accordance with a representative embodiment;

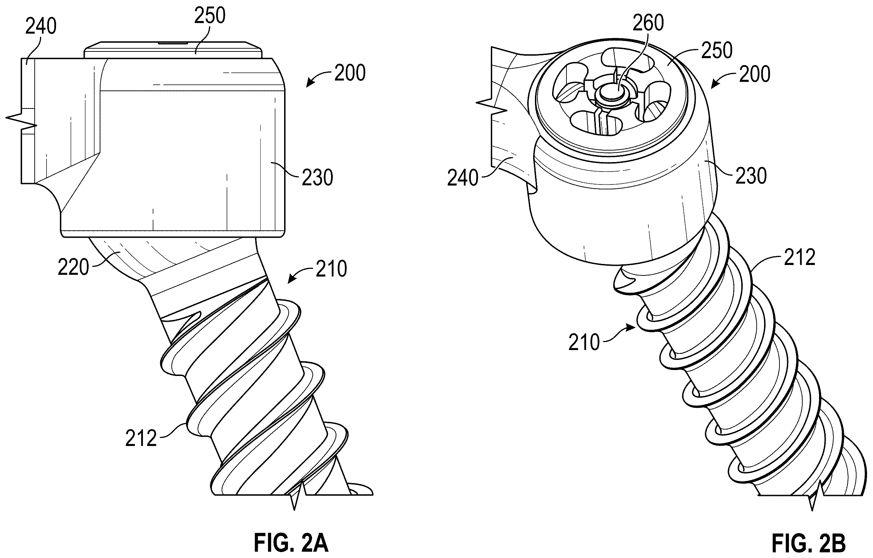

FIG. **2**B shows a perspective view of the rivet system of FIG. **2**A, in accordance with a representative embodiment;

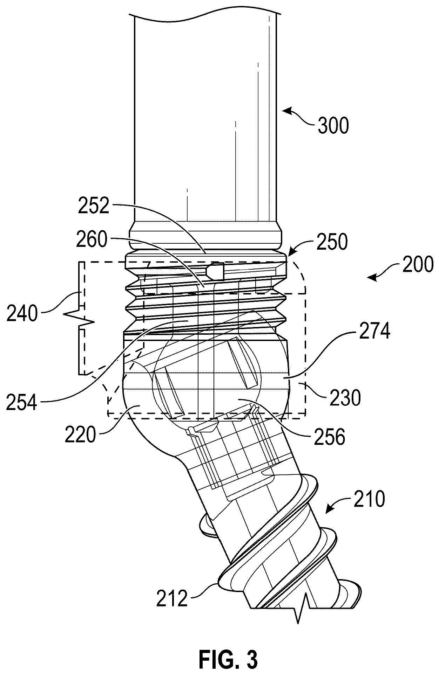

FIG. **3** shows a transparent elevation view of a rivet system including an applicator, in accordance with a representative embodiment;

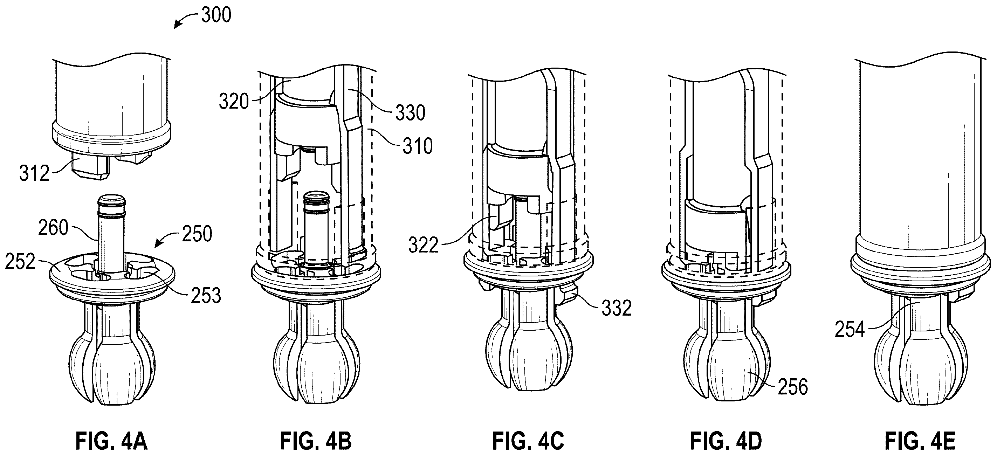

FIGS. **4**A-**4**E show perspective views (transparent in the case of FIGS. **4**B-**4**D) of various configurations of an applicator used in connection with a rivet, in accordance with a representative embodiment;

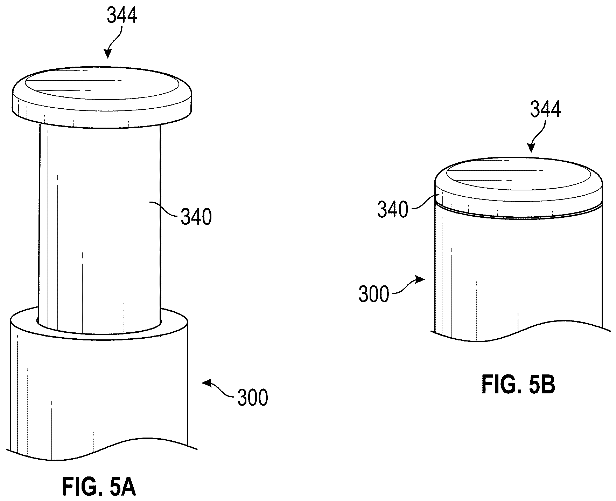

FIGS. **5**A and **5**B show perspective views of various configurations of an applicator's actuator, in accordance with a representative embodiment;

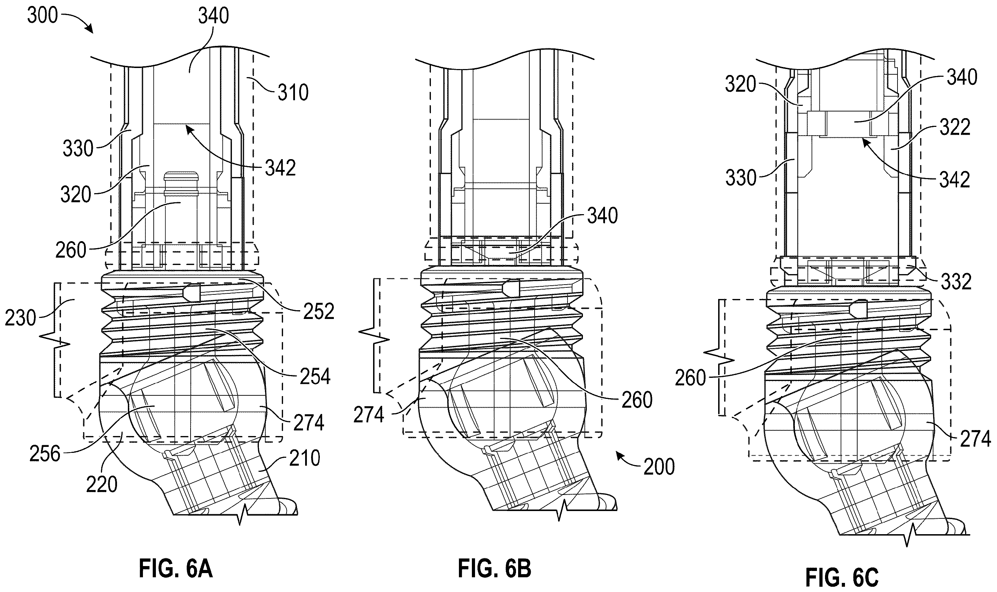

FIGS. **6**A-**6**C show transparent elevation views of a rivet system including an applicator in various configurations during a process of inserting a plug into a rivet, in accordance with a representative embodiment;

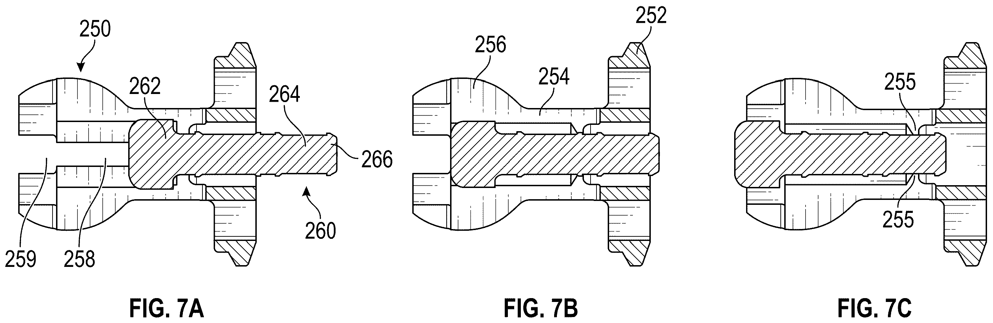

FIGS. **7**A-**7**C show cross-sectional cutaway elevation views of various configurations of a plug inserted into a rivet, in accordance with a representative embodiment;

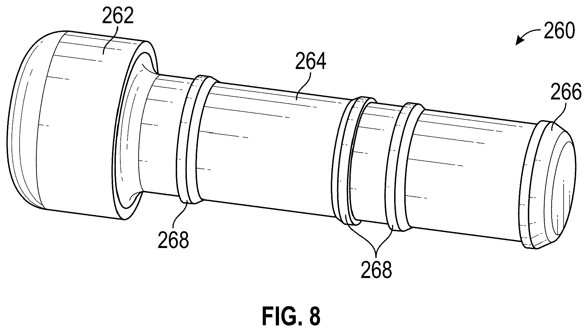

FIG. **8** shows a perspective view of a plug, in accordance with a representative embodiment;

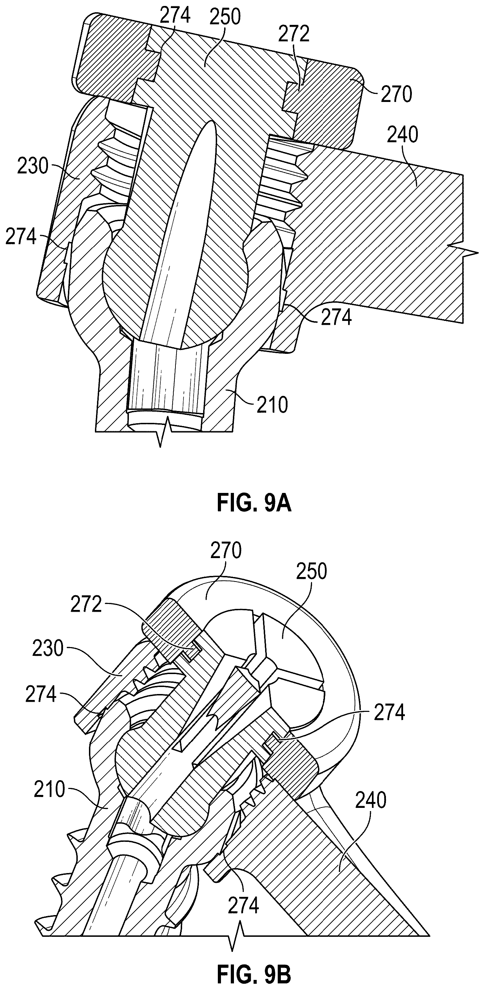

FIG. **9**A shows a perspective cutaway view of a rivet system with a collar, in accordance with a representative embodiment;
FIG. **9**B shows a perspective cutaway view of an alternative rivet system with a collar, in accordance with a representative embodiment;

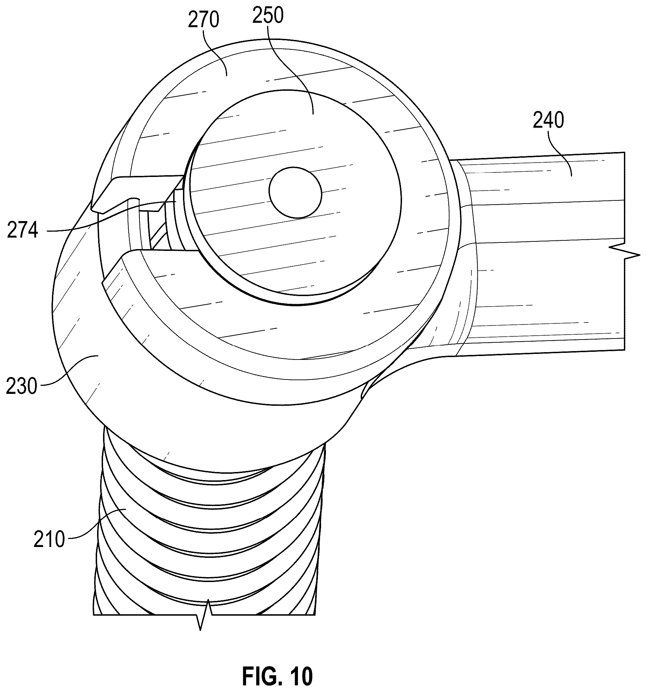

FIG. **10** shows a perspective view of a rivet system with a collar, in accordance with a representative embodiment;

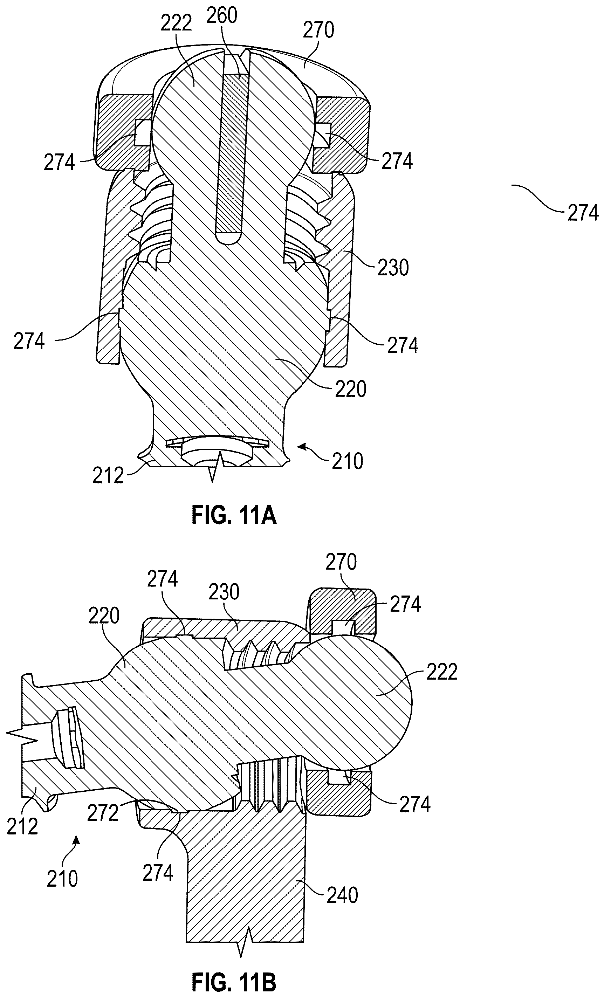

FIG. **11**A shows a perspective cutaway view of a rivet system with an anchor having a projection, in accordance with a representative embodiment;
FIG. **11**B shows a cross-sectional view of the rivet system of FIG. **11**A, in accordance with a representative embodiment;

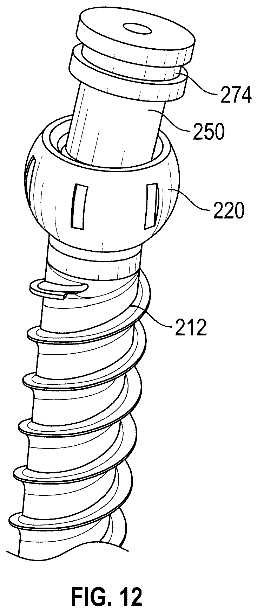

FIG. **12** shows a rivet coupled to an anchor, in accordance with a representative embodiment; and

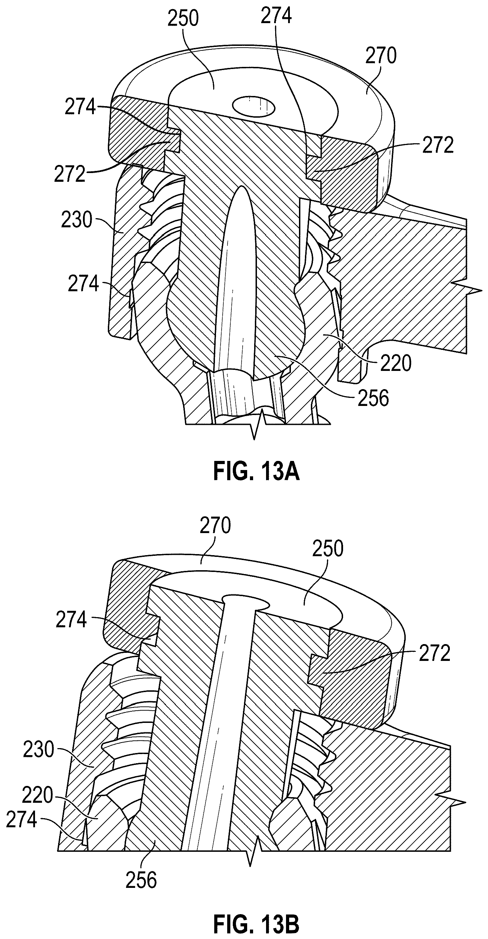

FIGS. **13**A and **13**B show a rivet system with a collar, in accordance with representative embodiments.

### DETAILED DESCRIPTION

A description of embodiments will now be given with reference to the Figures. It is expected that the present systems and methods may take many other forms and shapes, hence the following disclosure is intended to be illustrative and not limiting, and the scope of the disclosure should be determined by reference to the appended claims.

Many implants utilize set screws or similar mechanisms to hold various components together, such as to attach an implant body to an anchor. Unfortunately, in some traditional systems, the act of tightening a set screw causes potential energy to be stored in the tightened construct. The set screw is often retained in its tightened, high-energy position only through friction. Accordingly, when the system is exposed to external forces (e.g., vibration, loading, interaction with the system's environment, etc.), the stored potential energy in the set screw may be released, causing the set screw to loosen and compromising the integrity of the entire system (e.g., creating a risk of the screw head becoming detached from the coupler, or decreasing the rigidity and fidelity of the screw). Similar instances of loosening have been observed in many types of implants, wherever set screws or similar components are used.

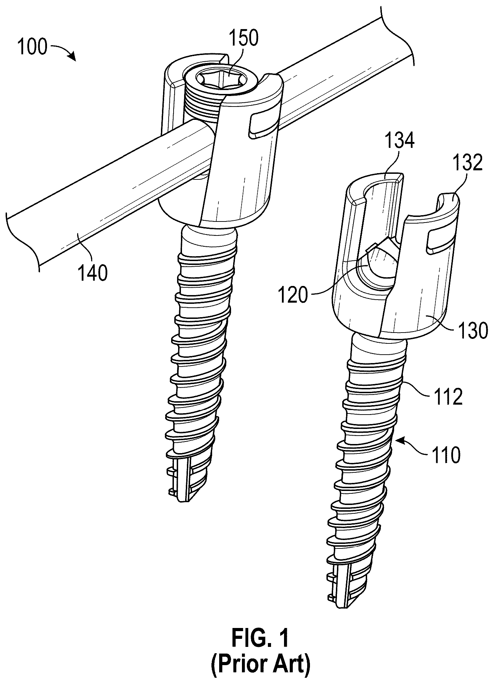

As an example, FIG. **1** shows a prior art pedicle screw system **100,** in which a rod **140** is retained within a tulip **130.** The tulip has a lower opening for receiving a screw **110** for connecting to a bone, organ, or another part of a patient's body. A screw head **120** of the screw is retained within the tulip, with a threaded rod **112** extending out of a bottom of the tulip. The rod is retained between a first face **132** and a second face **134** of the tulip and a set screw **150** is tightened in place (within the tulip, sealing in the rod) using threading on the first and second faces between the faces of the tulip. This system locks the rod in place, but the set screw is vulnerable to loosening-creating a risk that the entire system may become unstable or fail. Accordingly, the system may be improved by implementing some or all of the features discussed below.

Referring now to FIGS. **2**A and **2**B, according to some embodiments of the systems and methods disclosed herein, a rivet system **200** for providing a durable implant, and for securely attaching an anchor to an implant body, is provided. Although the rivet system may include any component suited to increasing the durability and integrity of an implant, or for providing a secure attachment between an anchor and an implant body, some embodiments of the rivet system include one or more anchors **210,** one or more couplers **230,** one or more rivets **250,** and any other suitable component.

Although the anchor **210** can include any component capable of attaching, annealing, or otherwise anchoring another component to a bone, organ, muscle, or another portion of a patient's or an animal's body, in some embodiments, the anchor comprises one or more shafts **212** and one or more anchor heads **220.** Although the shaft may include any suitable component for interfacing with a body or a body part in connection with an implant, in some embodiments, the shaft is threaded (e.g., like a bolt or a screw). Nevertheless, in some embodiments, the shaft is unthreaded, or the shaft includes one or more screws, nails, bolts, grafts, frames, wires, lattices, channels, drains, or other components of anchors (e.g., as used with pedicle anchors). The shaft of some embodiments includes a sharpened tip, while the shaft of other embodiments includes a flat tip, another kind of tip, or no tip at all.

While the anchor head **220** can include any terminus of the anchor **210,** some embodiments of the anchor head have a broader width or diameter than a diameter of the shaft **212.** Although some embodiments of the anchor head include one or more screw heads, screw drives, nail heads, bolt heads, hex heads, or other heads that may interface with a coupler **230** or rivet **250,** and although some embodiments of the anchor head may be generally spherical, semi-spherical, pyramidal, conical, tubular, cuboid, polygonal, symmetrical, asymmetrical, or have any other geometry, some embodiments are substantially cup-shaped.

Some embodiments of the anchor head **220** include a socket formed within the anchor head. In some cases, the socket is configured to interface with a protrusion formed on the rivet **205** (e.g., a rivet head **256,** as discussed in more detail below). That said, in some cases, the socket interfaces with any suitable complementary component, such as a spherical component (e.g., forming a ball-and-socket joint), a planar component (e.g., forming a hinge joint), a cylindrical component (e.g., forming a fixed joint), or another component for forming any other type of joint (e.g., a synovial joint, a pivot joint, an elbow joint, a planar joint, a saddle joint, etc.). FIG. 3 shows an example of a suitable shape of an anchor head **220,** with a socket having a shape that is generally complementary to the rivet head **256** disposed therein.

Returning to FIGS. **2**A and **2**B, some embodiments of the rivet system **200** include one or more couplers **230** for coupling the anchor **210** to an implant body **240** (e.g., a rod, a mesh, a shunt, a plate, a prosthetic, a device, or any other suitable type of implant body). While the coupler may include any component suitable for making such a coupling, in some embodiments, the coupler includes a housing having a channel extending therethrough. In some embodiments, the housing is integrally connected with the implant body, whereas in some embodiments the housing is permanently, semi-permanently, temporarily, or selectively attached to the implant body (e.g., through one or more adhesives, mechanical connectors, chemical bonds, or other connective means).

Some embodiments of the rivet system **200** include one or more rivets **250.** In this regard, the term "rivet" is not limited to a traditional rivet with a head that is configured to be pounded, compressed, or pressed into place. Rather, the term "rivet" means any component configured to secure the anchor **210** to the coupler **230.**

Some embodiments with an example of a rivet **250** are shown in more detail FIGS. **3-4**E and **6-7**C. In this regard, the rivet may include one or more: set screws; spring washers; adhesives (e.g., poly-methyl-methacrylate (PMMA) used as a thread locker to adhere threaded surfaces together); jam nuts; deformable plastics, such as polyethylene (PE) or ultra-high-molecular-weight polyethylene (UHMWPE), to increase an effective friction between the threaded surfaces; additional screws or check nuts to reduce backout; locking screws; locking washers; pins; friction fittings; or other components for attaching the anchor **210** to the coupler **230.** That said, in some embodiments, the rivet includes one or more rivet bases **252,** rivet necks **254,** and rivet heads **256** (or any of the foregoing in isolation). In some embodiments, the rivet base, rivet neck, and rivet head are included as a single, unitary construct, whereas, in some embodiments, the rivet is assembled from multiple separate components coupled together by any suitable means.

The rivet base **252** of some embodiments is configured to provide an interference fit (or another type of connection) with the coupler **230** such that one or more additional portions of the rivet **250** (e.g., the rivet neck **254** or the rivet head **256**) can be inserted completely or partially into the channel in the coupler while the rivet base remains completely or partially outside the coupler. In other embodiments, the rivet base does not remain outside the coupler, but engages in an interference fit or another type of connection within the coupler to prevent the rivet from falling or otherwise passing entirely through the channel of the coupler. Indeed, the rivet may include any assemblage or component allowing the rivet to be coupled to the anchor **210,** through the coupler, in a manner that retains the anchor in or with the coupler, thereby coupling the anchor to the coupler in a firm and secure manner. As an example, in some embodiments, all or a portion of the rivet base has a diameter (or width) that is larger than a diameter of the channel through the coupler, such that the rivet base is retained on the outside of the coupler (e.g., by abutting an exterior portion of the coupler) when another portion of the rivet is inserted into the channel (e.g., the rivet head, to be connected to the anchor).

Some embodiments of the rivet base **252** include an aperture that leads to a channel for receiving a plug **260** (as described in additional detail below). In some cases, the aperture includes a substantially circular perimeter, but in some cases the perimeter is oval, square, rectangular, triangular, polygonal, symmetrical, asymmetrical, or any other suitable shape corresponding to a cross section of any portion of the plug or otherwise configured to receive the plug (or to receive any portion of an applicator **300** configured to assist a practitioner in inserting the plug into the rivet **250**).

In some embodiments where the rivet **250** includes the rivet base **252,** the rivet base includes one or more rivet ports **253.** Although the rivet ports can include any feature providing a passage through the rivet base or any other feature suitable for coupling to an applicator **300,** in some cases, the rivet ports are evenly spaced around the aperture for receiving the plug **260,** and in some cases, the rivet ports are integrally formed with the aperture or joined with the aperture through channels, slits, or in any other suitable manner. In some embodiments, there are four rivet ports, but some embodiments include one, two, three, five, ten, or any other suitable number of rivet ports. In some embodiments, the rivet ports include a substantially oval diameter, but in some embodiments the diameter is circular, square, rectangular, triangular, elliptical, elongated, capsule shaped, symmetrical, asymmetrical, or any other suitable shape. In some embodiments the rivet ports are distinct from each other, whereas in some embodiments one or more rivet ports are joined together.

In some embodiments where the rivet **250** includes the rivet neck **254,** the rivet neck is configured to extend at least part of the way through the coupler **230** (see FIG. **3**). While the rivet neck may have any configuration suitable for extending through a coupler, in some embodiments, the rivet neck comprises a substantially cylindrical piece of material extending between the rivet base **252** and the rivet head **256.** In some embodiments, the rivet neck includes a channel for receiving a plug **260** formed therethrough. In some embodiments, the rivet neck is formed in multiple parts, such as two, three, four, or more neck members configured to be joined to one, two, three, four, or more rivet head members (as described in more detail below). Indeed, in some embodiments, the neck members are independent members that are not joined to each other, but are joined to the rivet base and run at any suitable angle (e.g., parallel, substantially parallel, within 20 degrees of being parallel or within any subrange thereof, or at any other suitable angle) to the rivet head. That said, some embodiments of the neck members are parallel or substantially (e.g., within 10 degrees or less of being parallel) to the rivet head and are joined together (in any suitable configuration or manner). As with the other components disclosed herein, the rivet neck is not confined to being any particular shape, but may take any shape or form that allows it to perform the function of joining the rivet head to the rivet base through the coupler.

In embodiments having a rivet head **256,** the rivet head may include any component or feature allowing the rivet **250** to interface with or attach to the anchor **210.** For example, in some embodiments, the rivet head includes a geometry that is complementary to the socket in the anchor head **220.** To illustrate, if the socket includes a bore having a substantially spherical shape, some embodiments of the rivet head have a substantially spherical geometry, thereby allowing for polyaxiality of the resulting joint, while retaining the anchor firmly connected to the rivet. If the socket includes a substantially cylindrical bore, the rivet head may have a substantially cylindrical geometry, thereby allowing for a monoaxial joint. Thus, different shapes of rivet heads may be desirable for use in different situations, depending on the desired degree of motion, if any, of the resulting joint. It is also worth noting that, as discussed in more detail below, some embodiments of the rivet head include a socket, while some embodiments of the anchor head include a protrusion configured to fit within the socket of the rivet head. Accordingly, while this disclosure frequently refers to the rivet head being configured to fit within the socket of the anchor, such disclosure should be interpreted as applying equally to a protrusion of an anchor head being configured to fit within a socket of a rivet head. Accordingly, the rivet head may have some or all the features of the anchor head (as discussed herein), and the anchor head may have some or all the features of the rivet head (as discussed herein).

Some embodiments of the rivet head include one or more rivet head members. In some embodiments, the rivet head members are attached to or integrated with one or more corresponding neck members. In some embodiments, due to a separation (or ability to separate) of the plurality of rivet head members, the rivet head has an expanded configuration and a compressed configuration (and in some embodiments, it has other configurations as well, such as a partially compressed configuration, an over-expanded configuration, or other configurations). As an example, in some embodiments, when the rivet head is in the expanded configuration some or all of the rivet head members are separated from each other by a gap, and when the rivet head is in the compressed configuration, some or all of the rivet head members are touching or abutting each other, or they are closer together (i.e., the gap is smaller) than when the rivet head is in the expanded configuration.

One example of a rivet **250** that falls within the scope of the rivet embodiments disclosed herein is shown in FIGS. 4A-4C. As shown in these Figures, and in accordance with some embodiments of the presently disclosed systems and methods, the rivet includes a rivet base **252** with a plurality of rivet ports **253** (in the case of the Figures, four) disposed radially around an aperture for a plug **260,** proximate to a perimeter of the rivet base. In some embodiments, a rivet neck **254,** including a plurality of neck members (in the case of the Figures, again four) extends from a bottom face of the base, with each neck member being formed integrally with the base, yet included as separate components that are not (in a default configuration) in contact with each other (thereby leaving a gap between each of the neck members and its immediate neighbor). A channel for receiving the plug extends therethrough in some cases. In some cases, each of the neck members connects to a rivet head member of a rivet head **256,** and each rivet head member corresponds to a neck member. According to some embodiments, the rivet head is substantially spherical, thereby being configured to fit within a substantially spherical bore of a socket. The rivet head as illustrated in the Figures is in an expanded configuration, with the rivet head members spaced apart from each of their neighbors by a gap, similar to the neck members. Indeed, in some embodiments, each gap extends all the way through a length of the neck and the rivet head (although in some embodiments, the gaps are separate or non-linear, or only one or neither of the rivet neck and the rivet head (or a portion of either) has a gap). In some embodiments, the rivet head members may be pushed towards each other by a force (e.g., a force of the rivet being pushed through the opening and into the socket of the anchor head **220**) in order to enter a compressed configuration.

As shown in FIGS. **2**B-**4**D and **6**A-**8**, the rivet system **200** of some embodiments includes a plug **260.** Although the plug can include any component (or components) of any size or shape suitable for locking the rivet head **256** in a particular configuration, in some embodiments the plug includes an object configured to be inserted through the aperture in the rivet base **252** and into the channel formed in the rivet neck **254** and rivet head.

In some embodiments, the plug **260** includes one or more plug heads **262,** and in some embodiments it includes one or more plug bodies **264.** In this regard, in some embodiments, the plug body includes any suitable configuration of a portion of the plug allowing the plug to be disposed within the channel of the rivet **250.** For example, in some cases a diameter of the plug body is less than a diameter of all or a portion of the channel. In embodiments in which the channel has a generally circular transverse cross section, the plug body may be generally cylindrical to fit snugly within the channel. However, in some embodiments, the plug body has a generally triangular, oval, square, rectangular, pentagonal, hexagonal, keyed, symmetrical, asymmetrical, or otherwise shaped transverse cross section (which, in some embodiments, corresponds with the transverse cross section of the channel, and in some embodiments simply fits within the channel having a cross section of a different shape).

According to some embodiments that include the plug head **262,** the plug head may include any feature for retaining the plug **260** within the rivet **250,** retaining the plug in a particular position within the rivet, locking the rivet in a particular configuration (e.g., the expanded configuration), or otherwise augmenting the functionality of the plug (in connection with the rivet or otherwise). For example, in some embodiments, the plug head has a diameter, width, or projection that is greater than a diameter (or width) of the plug body **264** (but, in some cases, the diameter (or width) of the plug head is the same as or less than the diameter (or width) of the plug body). Additionally, although the plug head may include any geometry or geometrical feature, in some cases, a tip of the plug head includes one or more rounded edges (e.g., to allow the plug to slide more easily towards a tip of the rivet head **256**)**.** In some embodiments, a base of the plug head (e.g., the side of the plug head disposed closest to the plug body) has one or more abrupt edges, allowing the base of the plug head to abut and catch on a catch **255** of the rivet **250** (as shown in FIGS. **7**A-**7**C). In some embodiments, the catch **255** provides an interference fit with the plug head, thereby preventing the plug from being removed from the rivet through the aperture of the rivet base **252.**

In some embodiments, the plug **260** is configured to retain the rivet **250** in a particular configuration (e.g., the expanded configuration, the over-expanded configuration) when the plug is in a certain position. For example, with further respect to FIGS. 7A-7C, in some embodiments the plug head **262** may shift between positions (e.g., by moving the plug). In some cases, the plug has a first position (e.g., where the plug head is located within the channel of the neck, or at a position closest to the base, as shown in FIG. **7**A). In some cases, the plug has a second position whereby the plug head is located within a first cavity **258** of the rivet head **256** (as shown in FIG. **7**B). In some cases, the plug has a third position whereby the plug head is located within a second cavity **259** of the rivet head (as shown in FIG. **7**C). In some embodiments, a diameter of the second cavity is larger than a diameter of the first cavity.

In some embodiments, when the plug is in the first position, the plug head does not lock the rivet in the expanded configuration (e.g., the rivet head members can be flexed toward one another, thereby decreasing the diameter of the rivet head and allowing the rivet head to be inserted into or removed from the socket of the anchor **210**)**.** In some embodiments, when the plug is in the second position, the plug head does lock the rivet in the expanded (or over-expanded) configuration, thereby preventing the rivet from being inserted into or removed from the socket of the anchor. Accordingly, in some embodiments, the rivet may be inserted into the socket while the plug is in the first position, then the plug may be shifted to the second position, thereby locking the rivet in the expanded configuration, such that the rivet head is then fixed in place within the socket of the anchor. In some embodiments, when the plug is in the third position (e.g., the plug head is disposed within the second cavity of the rivet head), the plug head does not lock the rivet head in a specific configuration. Accordingly, in some cases, the plug head may be pushed farther into the rivet to unlock the rivet head, thereby once again allowing the rivet head to be removed from (or inserted into) the socket of the anchor.

In some embodiments, the plug includes a plug end **266.** While the plug end may be any shape and have any configuration, in some embodiments, the plug end (or a portion thereof) has a larger diameter (or width) than the plug body **264.** In some cases, the plug end has a diameter (or width) approximately equal to or equal to the diameter (or width) of the plug head **262.** In some embodiments, the plug end is configured to form an interference fit (for example, through the use of one or more rings, protuberances, ridges, or other features) with the catch **255** to prevent the plug from being pushed too far through the rivet **250** such that it falls out of (or completely passes through) the rivet head **250** (e.g., to ensure that the plug cannot move beyond the third position).

As shown in FIG. **8****,** some embodiments of the plug include one or more ridges **268** or other features (such as protuberances, recesses, catches, and/or any other suitable features that are configured to help maintain the plug in one or more positions with respect to the rivet). While the ridges may be located at any position or positions along the plug body **264** (or any other portion of the plug **260**), in some cases the ridges are configured to retain the plug in preferred states (e.g., to generally shift the plug toward or retain the plug in one or more of the first position, the second position, and the third position). In some embodiments, the ridges are configured to provide tactile feedback to a healthcare provider performing an operation utilizing the systems or methods described herein. As an example, in some embodiments the ridges are configured to catch on the catch **255**-in some cases, not necessarily to the point of preventing the ridges from moving past the catch when force is applied to the plug, but instead to the point of preventing unintentional movement of the plug between positions. Moreover, in some cases the ridges are configured to allow an operator to feel a click, a vibration, or other tactile feedback when a ridge slides past the catch.

Although the ridges may include any configuration or component suitable for accomplishing one or more of the foregoing functions, in some embodiments, one or more of the ridges has a generally annular configuration, surrounding the plug body **264** with a raised (or sunken) ring. In some cases, one or both sides of the ring includes a chamfered surface. Where only one side of the ring includes the chamfered surface, it may be easier to slide the chamfered side with the chamfered surface past the catch **255** than to slide the side without a chamfered surface past the catch. Accordingly, in some embodiments, the ridges are configured to make disposition of the plug within one or more positions easier (e.g., within the second position, locking the rivet head **250** in the expanded configuration).

In some embodiments, the plug **260** has one or more fins or guide members to help retain the plug within the channel of the rivet **250.** For example, the fins may be disposed between the gaps between neck members or rivet head members.

In some cases, the rivet head **256** may be used without the plug **260,** in which case a retention force of the rivet head within the socket of the anchor **110** will be increased by the amount of force required to remove the rivet **250.** Where the rivet head is used with the plug, however, the retention force of the construct may be generally increased to that of the failure load of the rivet.

As shown in FIGS. **9**A-**10**, in some embodiments of the disclosed systems, the rivet **250** includes a collar **270.** The collar may include any suitable component for creating a strong coupling between the rivet at the coupler **230.** For example, in some embodiments, the collar includes a ring, a partial ring, a C-clamp, an E-clamp, a gasket, a washer, a nut, an adhesive, a welding, or any other component configured to couple both to the rivet and to the coupler. As shown in FIG. **10****,** some embodiments of the collar include a clamp that attaches to the rivet via a snap-fit (e.g., configured to slide onto the rivet radially, from an axis that is perpendicular to a longitudinal axis of the rivet, from a longitudinal axis, or in any other suitable manner), although some embodiments include a threading for screwing onto the rivet (or the coupler) or another attachment mechanism. In some embodiments, the collar augments the functionality of the rivet base **252,** and in some embodiments, the collar replaces the rivet base. In some embodiments, the collar attaches to, replaces, or works in connection with the rivet neck **254** or the rivet head **256.** Some embodiments of the collar include a notch **272** (or a groove, such as **274** of FIGS. **11**A-**11**B), or another feature for increasing its retaining capacity. In some embodiments with the notch, the notch interfaces with one or more corresponding grooves formed in the coupler.

Although any of the embodiments discussed herein can include a collar **270** used with (or instead of) a rivet **250,** embodiments with a collar may be particularly useful where it is desired for the rivet to have a more cylindrical configuration (as shown in FIGS. **9**A-**10**). In this regard, some embodiments of the rivet may have a diameter that is less than the diameter of the passage formed through the coupler **230.** In some such embodiments, the collar prevents the rivet from falling through the coupler.

Although different types of connections are advantageous in different circumstances, a cylindrical rivet may be desirable where a high degree is tensile strength is required. Indeed, in some embodiments with a cylindrical connection, a fitting requiring hundreds of pounds of force to connect (e.g., 100-300 lbs or 45.36 kg-136.10 kg, or any other suitable amount) may require a force of thousands of pounds to disconnect (e.g., 1,000-10,000 lbs or 453.59 kg-4,535.92 kg, or any other suitable amount). In contrast, some spherical connections require approximately the same amount of force to disconnect as they do to connect (e.g., about 1,000 lbs or 453.59 kg to connect, and about 1,000 lbs or 4,535.92 kg to disconnect). That said, many of the embodiments discussed herein can greatly increase the amount of force required to disconnect both spherical and cylindrical connects, along with any other type of connection. As a few examples, a fitting requiring anywhere between 10 lbs-1,000 lbs or 4.54 kg-453.59 kg (or any subrange thereof) of force to connect can require anywhere between 15 lbs-20,000 lbs or 6.80 kg-9,071.85 kg (or any subrange thereof) of force to disconnect, or even withstand a greater force (up to as much as the maximum strength of the failure loads of the materials used).

As shown in FIGS. **11**A and **11**B, in some embodiments, the rivet head (e.g., in isolation, as used in connection with other parts of the rivet, or in any other configuration as discussed herein or as otherwise useful for coupling an anchor to a coupler) includes one or more collars **270.** In some embodiments, the collar includes one or more sockets, grooves **274,** or any other features allowing the collar to receive one or more protrusions **222** of an anchor **210.**

While a groove **274** formed in a collar **270** may have any characteristics suitable for forming or strengthening a coupling with a protrusion **222** of an anchor head **220,** in some embodiments, the groove is formed in an inner face of the collar in a 360 degree (or partial) arc. In some embodiments, the groove extends only part way around the inner face of the collar. In some embodiments, the geometry of a cross section of the groove is substantially square, although the cross section of the groove may also be rectangular, triangular, circular, semi-circular, irregular, or any other shape. Some embodiments of the groove (as shown in FIG. 11B) allow for the protrusion **222** of the anchor head **220** to be disposed within the collar at an angle. Indeed, while the protrusion may be perpendicular to a radius of the collar, in some embodiments, the protrusion is disposed within the collar at an angle (e.g., such that the anchor is not perpendicular to the radius of the collar). In some embodiments, the angle of the protrusion within the collar is locked in place, whereas in some embodiments the protrusion is allowed a range of motion (e.g., polyaxial, biaxial, etc.) within the collar. In some embodiments, the protrusion includes processes configured to be disposed within the groove.

As with some other embodiments herein, in some embodiments in which the anchor includes the protrusion, the anchor has an anchor shaft **212** and an anchor head **220,** with the protrusion being formed on the anchor head. Thus, in some such embodiments-like some other embodiments discussed herein-the coupler **230** is configured to receive the anchor head, and the rivet (including the collar) is configured to attach to the anchor head in order to couple the anchor to the coupler (and, transitively, to the implant body **240**). In some embodiments, the protrusion of the anchor head has a passage for receiving one or more plugs **260.** Accordingly, in some embodiments, the plug may be inserted (e.g., using an applicator, as discussed below, or in any other suitable manner) through the rivet and into the protrusion of the anchor head. As with some other embodiments discussed herein, the protrusion of the anchor head can also be retained within the socket of the rivet head (or the retention strength may be increased) in any other suitable manner, including through use of: one or more additional mechanical parts (e.g., screws, bolts, etc.); adhesives; staples, rivets, or other similar fasteners; and any other suitable components.

FIGS. **12-13B** further highlight the versatility of some embodiments of the disclosed systems and methods. For example, FIG. **12** shows how a rivet **250** may be disposed within an anchor head **220** at any angle relative to the anchor shaft **212.** FIGS. **13**A and **13**B illustrate how a socket of an anchor head **220** receives a rivet head **256** in some embodiments, and how a collar **270** coupled to a rivet **250,** preventing the rivet from being puller through a coupler **230** as a result of the connection to the anchor head.

With reference to FIGS. **3-6**C, some embodiments of the disclosed systems and methods include an applicator **300.** In this regard, the applicator may include any component suited to assist a person in attaching the rivet **250** to the anchor **110** through the coupler **130.** That said, in some embodiments, the applicator includes one or more applicator casings **310,** which may include any component configured to be held by a person assembling the rivet system **200,** or to contain one or more other portions of the applicator. Although the applicator casing is not limited to any particular shape or configuration, in some embodiments the casing includes an elongated, generally cylindrical component (somewhat similar to a pen or a pipette). In some embodiments, the applicator casing includes one or more attachment features for interfacing with the rivet **250,** such as one or more attachment nubs **312** configured to be inserted into a rivet port **253.**

In some embodiments, the applicator **300** includes an applicator body **320,** which in some cases is housed within the applicator casing **310.** Some embodiments of the applicator body are slidable within the casing. Generally speaking, the applicator body may include any component necessary or useful for the function of the applicator (e.g., applying the rivet **150** to the socket of the anchor **110**)**.** For example, in some embodiments, the applicator body includes one or more stabilizers **322** for stabilizing a connection between the applicator and the rivet base **252.** As an illustration of this, some embodiments of the stabilizer insert into one or more rivet ports **253** of the rivet base (e.g., the rivet ports adjacent to the rivet ports into which the attachment nubs **312** of the casing are inserted).

In some embodiments, the applicator **300** includes one or more retainers **330** for connecting the applicator to the rivet **250,** such as via one or more retaining anchors **332.** Although the retaining anchors can include any clamps, clasps, hooks, snaps, screws, adhesives, pins, protrusions, staples, or other fasteners suitable for establishing such a connection, in some embodiments, the retaining anchors include one or more hooked or notched ends for inserting through a rivet port **253** and selectively establishing an interference fit. In some cases, the retaining anchors are configured to insert into the same rivet ports as the stabilizers **322.** In some embodiments, the retaining anchors are configured to snap in place, and in some embodiments the retaining anchors are configured to engage (and disengage, in some cases) through rotating the retainer with respect to the rivet.

In some embodiments, the retainer **330** can be selectively disengaged from the rivet. In some cases, the retainer may be selectively disengaged by manually disengaging the retaining anchors (e.g., but pushing in on a pair of retaining anchor hooks). In some embodiments the retainer includes a disengaging mechanism (e.g., a lever, a button, a switch, a knob, or another mechanical or electronic feature that may be activated by a user, causing the retainer to disengage from the rivet. In some cases, the retainer is configured to automatically disengage from the rivet after application of the rivet (e.g., after insertion of the plug **260**)**.**

As shown in FIGS. **4**A-**4**E, in some embodiments the applicator **300** is configured to couple to the rivet **250,** after which the applicator body **320** is configured to slide into place, thereby positioning the actuator (as further discussed below) in a proper position to insert the plug **260** the desired position. The applicator body may be configured to slide along the applicator casing **310** in any suitable manner, such as by using a slider disposed on the exterior of the housing, by flipping a switch, by pressing a button, or through any other means. With the applicator coupled to the rivet and the applicator body in place, a practitioner is free to insert the rivet into the socket of the anchor **110** (and may do so by holding onto and manipulating the applicator, thereby simplifying what otherwise might be a highly difficult task of properly positioning the rivet within a patient).

As shown in FIGS. **5**A-**6**C, some embodiments of the applicator **300** include an actuator **340.** Although the actuator may interface with the applicator in any suitable manner, in some embodiments the actuator is housed within the applicator casing. Although the actuator may include any mechanical feature, motor, electronic component, feedback mechanism, or any other component that may be helpful in connection with the application of the rivet **250,** in some instances the actuator includes a mechanical piston for pushing on the plug **260** to move it from the first position (as described above) to the second position (to lock the rivet in the expanded configuration, thereby firmly securing the rivet head **256** to the anchor **210.** For example, a user may mechanically activate (e.g., by pushing, pulling, sliding, turning, etc.) a user end **344** of the actuator, causing a rivet end **342** of the actuator to mechanically move the plug (e.g., from the first position to the second position, from the second position to the third position). In some embodiments, the actuator is configured to operate in a retrograde direction as well (or a separate actuator is provided for operating in a retrograde direction), thereby allowing for pulling the plug from the third position to the second position or from the second position to the first position. In some embodiments, the actuator includes a switch (or another toggling feature) for changing the operation of the actuator from an anterograde direction to a retrograde direction (and vice versa). In some embodiments, the applicator simply includes a rod (which may be cylindrical, square, or any other shape), whereby pushing on the user end naturally causes the rivet end to move, thereby pushing on the plug.

Although, in some cases, the user end is configured to move the plug to the second and third positions, in some cases, the user end is modified such that one type of user end is configured to push the plug into the second position but not into the third position (e.g., the user end comprises a wide or keyed user end that prevents it from pushing the plug to the third position, the user end defines a shallow recess that will not permit the user end to push the plug past the second position, or the user end is otherwise configured to move the plug to the second position but not the third position). In some cases, however, the user end is sized, shaped, or otherwise configured to move the plug to both the second position and the third position.

In some implementations, any or all of the components of the applicator are independently slidable within the applicator casing, thus allowing any or each to be manipulated independently of the others (e.g., disconnecting the retainer, withdrawing the actuator while the retainer is still connected, etc.).

With respect FIGS. **2**A-**8**, Some embodiments of the disclosed systems and methods include a method of securing an anchor **210** to an implant body **240.** In this regard, the method may include any technique suitable for using any of the systems discussed above, or any components thereof, to secure an anchor to an implant body. Moreover, one or more of the various portions of the described method can be omitted, substituted, performed in parallel, performed in series, repeated, modified, reordered, or changed in any other suitable manner. Nevertheless, the method can, more specifically, include any of the following features:

According to some embodiments, the method includes obtaining a coupler **230** that is coupled to (or configured to couple to) the implant body **240.** In some embodiments, the method includes coupling the coupler to the implant body. In some embodiments, the method includes forming a passage through the coupler.

According to some embodiments, the method includes inserting an anchor head **220** of the anchor **210** into the coupler (e.g., into the passage), in some cases, retaining an anchor shaft **212** outside the coupler. In some embodiments, the method further includes fixing the anchor shaft in place, allowing it to move freely, or allowing it to follow a limited set of specific movements, as may be desirable for the implant in question.

Some embodiments of the method include inserting a rivet head **256** of a rivet **250** into a socket of the anchor head **220,** thereby attaching the anchor **210** to the coupler **230.** Some embodiments include, prior to inserting the rivet head, shifting the rivet head from an expanded configuration to a compressed configuration in order to allow the rivet head to fit through an opening to the socket of the anchor head. Some embodiments include, after inserting the rivet head into the socket of the anchor head, returning the rivet head to the expanded configuration. Some embodiments include locking the rivet head in the expanded configuration. Some embodiments include inserting a plug **260** in order to lock the rivet head in the expanded configuration.

According to some embodiments, the method includes using an applicator **300** to assist with any of the foregoing. As an example, some embodiments of the method include attaching the applicator to the rivet **250.** In some embodiments, this includes inserting one or more of an attachment nub **312,** a stabilizer **322,** and a retaining anchor **332** into one or more rivet ports **253.** In some embodiments, the method includes rotating an applicator casing **310** to cause a retainer **330** to engage with the rivet **250.**

In some embodiments, the method includes activating an actuator **340** of an applicator **300.** In some embodiments, activation of the actuator causes insertion of the plug **260** into the rivet **250.** In some embodiments, the method includes inserting the plug into the rivet **250** to lock the rivet head **256** in the expanded configuration. Some embodiments of the method include pushing the plug farther into the rivet head in order to unlock the rivet head, thereby allowing it to shift back to the compressed configuration so the rivet head can be removed from the socket of the anchor head.

In some embodiments, whether the rivet head **256** is used with or without the plug **260,** the method optionally includes deforming the rivet **250** (e.g., through pounding, crimping, expanding, bending, pressing, melting, or otherwise altering the rivet) to further increase the retention force within the socket.

In some embodiments, the method includes securing the anchor **210** to a patient's body (e.g., bone, organ, muscle, tissue, or other body part) prior to inserting the rivet head **256** into the socket. In some cases, securing the anchor to a patient is performed concurrently with or after inserting the rivet head into the socket.

Some embodiments of the disclosed systems and methods include a method for forming the systems discussed herein. In this regard, the systems and discussed herein, and each and every component thereof, can be made in any suitable manner, from any suitable material. Indeed, in some embodiments, various components are die-cast, injection molded, 3D printed, sculpted, forged, laser cut, drilled, extruded, formed through computer numerical control, additive manufacture, or constructed using any other technique. Moreover, the various components discussed herein can be formed of any suitable material or materials, including plastic, metal, alloy, wood, ceramics, mesh, resin, carbon fiber, fiberglass, biological material, infection-resistant material, polymers, wax, or any other substance.

The systems and methods described herein can be modified in any suitable manner. For example, in some embodiments, the gaps between the rivet head members are filled or partially filled with a compliant material configured to bias the rivet head members toward the expanded configuration. In some embodiments, a switch or other mechanical or electronic feature is integrated into the rivet **250,** the applicator **300,** or another component in order to toggle the rivet head **256** between the expanded and compressed configurations. In some implementations, the applicator includes a separate attachment or is otherwise configured to remove the plug 260 or push the plug farther into the rivet head. In some embodiments, the order of the various parts of the methods discussed herein can be rearranged.

Another suitable modification is that instead of the protraction expanding to increase the retention force within the socket, the socket may be contracted. Indeed, in some embodiments, the socket is plastically deformed or otherwise constricted (e.g., through bending, pounding, melting, pressing, binding, wrapping, shrinking, crimping, or otherwise adapting the socket) in order to securely retain the protraction.

While the systems and methods described herein may provide many advantages over existing systems and methods, according to some embodiments, the systems and methods herein provide an advantage at least in that the rivet system **200** greatly increases a removal force required to separate the anchor **210** from the coupler **230** due to the diameter of the rivet head (in the expanded configuration) exceeding the diameter of the opening to the socket, thereby preventing the anchor from being separated from the socket. Additionally, in some embodiments, the rivet system may increase a friction between the anchor head **220** and the coupler **230** such that the removal force (or a force necessary to cause relative motion between the implant body **240** and the anchor **110**) is increased.

As the systems and methods disclosed herein are compatible with one another, the systems discussed herein can be used in practicing the methods disclosed herein, and vice versa. Accordingly, the method may further include implementing, exercising, or otherwise using any of the components discussed herein for any of their stated or intended purposes, as reasonably predictable and understood by a person of ordinary skill in the art. The systems disclosed herein can be made in any suitable manner, and they may be used in any way consistent with their operational capabilities. Moreover, in some cases, any particular element or elements of any apparatus-or portion or portions of any method-disclosed herein can be omitted.

As used herein, the singular forms "a", "an", "the" and other singular references include plural referents, and plural references include the singular, unless the context clearly dictates otherwise. For example, reference to an actuator includes reference to one or more retractors, and reference to rivet head members includes reference to one or more rivet head members. In addition, where reference is made to a list of elements (e.g., elements a, b, and c), such reference is intended to include any one of the listed elements by itself, any combination of less than all of the listed elements, and/or a combination of all of the listed elements. Moreover, the term "or" by itself is not exclusive (and therefore may be interpreted to mean "and/or") unless the context clearly dictates otherwise. Furthermore, the terms "including", "having", "such as", "for example", "e.g.", and any similar terms are not intended to limit the disclosure, and may be interpreted as being followed by the words "without limitation".

In addition, as the terms "on", "disposed on", "attached to", "connected to", "coupled to", etc. are used herein, one object (e.g., a material, element, structure, member, etc.) can be on, disposed on, attached to, connected to, or otherwise coupled to another object-regardless of whether the one object is directly on, attached, connected, or coupled to the other object, or whether there are one or more intervening objects between the one object and the other object. Also, directions (e.g., "front", "back", "on top of", "below", "above", "top", "bottom", "side", "up", "down", "under", "over", "upper", "lower", "lateral", "right-side", "left-side", "base", etc.), if provided, are relative and provided solely by way of example and for ease of illustration and discussion and not by way of limitation.

The described systems and methods may be embodied in other specific forms without departing from their spirit or essential characteristics. The described embodiments, examples, and illustrations are to be considered in all respects only as illustrative and not restrictive. The scope of the described systems and methods is, therefore, indicated by the appended claims rather than by the foregoing description. All changes that come within the meaning and range of equivalency of the claims are to be embraced within their scope. Moreover, any component and characteristic from any embodiments, examples, cases, implementations, and illustrations set forth herein can be combined in any suitable manner with any other components or characteristics from one or more other embodiments, examples, cases, implementations, and illustrations included herein.

Arrangements of the invention are further described in the following numbered list of clauses.

Clause 1. A system for securely attaching an anchor to an implant body, the system comprising:
the anchor comprising an anchor head;
a coupler configured to couple to the implant body, and further configured to receive the anchor;
a rivet;
a projection formed on at least one of the anchor head and the rivet; and
a socket formed in at least one of the anchor head and the rivet, wherein the socket is configured to receive the projection.

Clause 2. The system of clause 1, wherein the anchor comprises the socket, wherein the projection comprises a rivet head formed on the rivet, and wherein when the socket receives the rivet head, the anchor is joined to the coupler.

Clause 3. The system of clause 2, wherein the rivet further comprises:
a rivet base configured to remain exterior to the coupler; and
a rivet neck configured to extend through at least a portion of the coupler.

Clause 4. The system of clause 3, wherein the rivet base further comprises a rivet port for receiving a portion of an applicator configured to assist a user in coupling the rivet head to the anchor through the coupler.

Clause 5. The system of clause 2, wherein the rivet head comprises a plurality of rivet head members, wherein when the rivet head is in an expanded configuration the rivet head members are separated from each other by a gap.

Clause 6. The system of clause 5, wherein the socket comprises an opening, and wherein the rivet head comprises a size and shape such that rivet head does not fit through the opening when the rivet head is in the expanded configuration.

Clause 7. The system of clause 6, wherein the rivet head members are configured to be selectively biased towards each other, thereby reducing the size the gap, thereby shifting the rivet head to a compressed configuration, wherein the rivet head can fit through the opening when the rivet head is in the compressed configuration.

Clause 8. The system of clause 7, wherein the rivet head is biased toward the expanded configuration.

Clause 9. The system of clause 7, further comprising a plug configured to be inserted into the rivet to retain the rivet head in the expanded configuration.

Clause 10. The system of clause 9, wherein the plug comprises a plug body and a plug head, wherein the plug head is configured to retain the rivet head in the expanded configuration when the plug head is positioned within a first cavity of the rivet head.

Clause 11. The system of clause 10, wherein rivet head comprises a second cavity proximate the first cavity, and wherein when the plug head is positioned within the second cavity, the plug does not prevent the rivet head from shifting to the compressed configuration.

Clause 12. The system of clause 9, further comprising an applicator configured to assist a user in coupling the rivet head to the anchor through the coupler.

Clause 13. The system of clause 12, wherein the applicator comprises: a retainer configured to selectively secure the applicator to the rivet; and an actuator configured to insert the plug into the rivet head.

Clause 14. The system of clause 1, wherein the projection is formed on the anchor head, wherein the rivet head comprises a collar, and wherein the socket is formed in the collar.

Clause 15. A method of securing an anchor to an implant body, the method comprising: obtaining a coupler configured to couple to the implant body; inserting an anchor head of the anchor into the coupler; inserting a rivet head of a rivet into a socket of the anchor head, thereby attaching the anchor to the coupler.

Clause 16. The method of clause 15, wherein the rivet head has an expanded configuration, and wherein the rivet head is configured to remain securely within the socket when the rivet head is in the expanded configuration.

Clause 17. The method of clause 16, wherein the method further comprises inserting a plug into the rivet to selectively lock the rivet head in the expanded configuration.

Clause 18. The method of clause 17, wherein the inserting a plug into the rivet comprises activating an actuator of an applicator.

Clause 19. The method of clause 18, wherein the actuator is configured to selectively couple to a base of the rivet.

Clause 20. A system for providing a durable implant, the system comprising:
an implant body coupled to a coupler;
an anchor configured to be secured to a body of a patient;
a rivet configured to insert at least partially into the coupler and attach to the anchor through the coupler, thereby securing the anchor to the coupler; and
an applicator configured to selectively attach to the rivet for facilitating attachment of the rivet to the anchor.

## Claims

1. An implant comprising:
an anchor comprising an anchor head;
a coupler that defines a receptacle configured to receive the anchor head; and
a groove formed around an inner perimeter of the receptacle, wherein a portion of the anchor head is configured to be received within the groove when the anchor head is disposed within the receptacle.

2. The implant of claim 1, further comprising a rivet configured to increase a strength of a connection between the receptacle and the anchor.

3. The implant of claim 1, wherein the anchor head defines a socket.

4. The implant of claim 2, wherein the anchor head defines a socket, wherein the rivet further comprises a projection configured to be received within the socket, wherein the projection is configured to have an expanded configuration and an unexpanded configuration, and wherein, in the expanded configuration, a portion of the projection is larger than an opening of the socket.

5. The implant of claim 4, wherein the projection defines a passage formed therein.

6. The implant of claim 5, further comprising a plug configured to be inserted into the passage to place the projection in the expanded configuration.

7. The implant of claim 6, wherein the plug comprises a plug body and a plug head, wherein the plug head has a greater diameter than the plug body.

8. The implant of claim 7, wherein the projection is configured to selectively be placed in the unexpanded configuration when the plug body is positioned in the passage, but the projection is configured to selectively remain in the expanded configuration when the plug head is positioned in the passage.

9. The implant of claim 1, wherein the groove is formed around an entirety of the inner perimeter of the receptacle.

10. The implant of claim 4, wherein the projection is configured to extend past the groove when the projection is disposed within the anchor head and the anchor head is disposed within the receptacle.

11. The implant of claim 7, wherein the projection comprises a first cavity and a second cavity that is proximate to the first cavity, and wherein when the plug head is positioned within the second cavity, the plug does not prevent the projection from shifting to the unexpanded configuration.

12. The implant of claim 1, wherein the groove is disposed in an internal chamber of the receptacle, away from an opening of the receptacle.

13. The implant of claim 1, wherein a height of the groove is less than half a height of the receptacle.

14. The implant of claim 1, wherein the groove comprises a shoulder having a generally right-angled corner.

15. The implant of claim 1, wherein the groove runs parallel to an outer perimeter of the receptacle.
